(19)

Europäisches Patentamt

European Patent Office

Office européen des brevets

(11) **EP 2 544 593 B1**

(12) **EUROPEAN PATENT SPECIFICATION**

(45) Date of publication and mention of the grant of the patent:
**31.12.2014 Bulletin 2015/01**

(21) Application number: **11711795.2**

(22) Date of filing: **08.03.2011**

(51) Int Cl.:
***A61B 8/00*** *(2006.01)*    ***A61B 8/06*** *(2006.01)*

(86) International application number:
**PCT/EP2011/053460**

(87) International publication number:
**WO 2011/110552 (15.09.2011 Gazette 2011/37)**

(54) **INITIALIZATION OF FITTING PARAMETERS FOR PERFUSION ASSESSMENT BASED ON BOLUS ADMINISTRATION**

INITIALISIERUNG VON ANPASSUNGSPARAMETERN FÜR DIE PERFUSIONSBEURTEILUNG BASIEREND AUF DER BOLUSVERABREICHUNG

INITIALISATION DES PARAMÈTRES DE RÉGLAGE POUR L'ÉVALUATION DE PERFUSION BASÉE SUR UNE ADMINISTRATION DE BOLUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.03.2010 EP 10155926**

(43) Date of publication of application:
**16.01.2013 Bulletin 2013/03**

(73) Proprietor: **Bracco Suisse SA**
**6928 Manno (CH)**

(72) Inventors:
• **FRINKING, Peter**
**CH-6928 Manno (CH)**

• **ROGNIN, Nicolas**
**CH-6928 Manno (CH)**
• **ARDITI, Marcel**
**CH-6928 Manno (CH)**

(74) Representative: **Pezzoli, Ennio**
**c/o Maccalli & Pezzoli S.r.l.**
**Via Settembrini 40**
**20124 Milano (IT)**

(56) References cited:
**WO-A1-2004/110279    WO-A1-2009/083557**
**WO-A2-2006/067201**

**Description**

Technical field

The solution according to one or more embodiments of the present invention relates to the diagnostic field. More specifically, this solution relates to diagnostic applications based on bolus administration of contrast agent.

Background of the invention

Contrast agents are commonly used in diagnostic applications, such as for the assessment of blood perfusion in contrast-enhanced ultrasound imaging applications. For this purpose, during an analysis process of a specific body-part of a patient, an ultrasound contrast agent (UCA) - for example, consisting of a suspension of phospholipid-stabilized gas-filled microbubbles - is administered to the patient. The contrast agent acts as an efficient ultrasound reflector, and it can be easily detected by applying ultrasound waves and measuring echo signals that are returned in response thereto. Since the contrast agent flows at the same velocity as red-blood cells in the patient, its detection and tracking in the body-part under analysis provides information about the corresponding blood perfusion.

Particularly, in a quantitative approach, the echo signal is recorded over time during the whole analysis process for each location of the body-part, and it is fitted by a parametric function using a best-fit optimization process. This optimization process generates a time-intensity function, which consists of an instance of the parametric function being defined by the best-fit values of its fitting parameters. The values of different perfusion parameters are calculated from the time-intensity function (such as a time to peak, a mean transit time, and the like); these perfusion parameter values are then used to characterize the corresponding location (for example, for detecting and identifying a lesion). Any perfusion parameter may be calculated from the echo signal over time that is obtained in a predefined Region of Interest (ROI) - with the perfusion parameter value that is then presented as a single value. Alternatively, any perfusion parameter may be calculated from the echo signal over time of each basic portion of the body-part individually; a parametric image is then generated by graphically visualizing the perfusion parameter values of the different basic portions of the body-part (preferably in a color-coded representation). The perfusion parameter values provide a quantitative assessment of the blood perfusion in the body-part (with the parametric images representing a spatial map of the perfusion parameter values throughout the body-part).

The contrast agent may be administered to the patient as a bolus (i.e., a single dose provided over a short period of time). The bolus administration is very simple, and it can be carried out by hand (for example, using a syringe); moreover, this requires a small amount of contrast agent. Different examples of quantitative analyses based on bolus administration are disclosed in WO-A-2006/108868, WO-A-2006/067201, WO-A-2009/083557, WO-A-2010/058014, and US-B-6,216,094, as well as in "Quantification of perfusion of liver tissue and metastases using a multivessel model for replenishment kinetics of ultrasound contrast agents - Martin Krix, Christian Plathow, Fabian Kiessling, Felix Herth, Andreas Karcher, Marco Essig, Harry Schmitteckert, Hans-Ulrich Kauczor, And Stefan Delorme, Ultrasound in Med. & Biol., Vol. 30, No. 10, pp. 1355-1363", and "A new method of analyzing indicator dilution curves, Cardiovascular Research - R.A.F. Linton, N.W.F. Linton and D.M. Band, vol. 30, pp. 930-938, 1995.

Particularly, WO-A-2009/083557 discloses as closest prior art for the present invention a method for detecting and quantifying targeted contrast agent that immobilizes on a specific target. For this purpose, the echo signal is fitted by an instance of a model function based on a combination of a circulation function (modeling the circulation of the contrast agent) and a dynamic immobilization function (modeling the immobilization of the contrast agent and the decay of its echo signal). In a specific implementation, the fitting operation is performed in two optimization steps. At first, an initial portion of the echo signal is defined up to a predefined multiple of its peak instant (when the echo signal reaches the maximum value thereof). The initial portion of the echo signal is fitted by an instance of the same circulation function alone, so as to determine the values of the corresponding fitting parameters (i.e., $A$, $m$ and $s$ in the case of a lognormal distribution function). This instance of the circulation function provides a good estimate of each signal (since only a small fraction of the contrast agent immobilizes at the beginning); moreover, the dynamic immobilization function depends on the circulating function (i.e., on its integral). Therefore, the same fitting parameters $A$, $m$ and $s$ of the model function may be initialized exactly to the values determined above (and constrained to vary during the corresponding optimization step within a predefined range thereof); the fitting of the echo signal by the model function may then be focused on the determination of its remaining fitting parameters alone (i.e., an immobilization parameter and a decay parameter).

Typically, the parametric function consists of a simple bolus function (for example, the lognormal distribution function), as used in classical indicator-dilution approaches, which is adapted to model the typical trend of the echo signal over time (with a wash-in followed by a wash-out of the contrast agent). However, the simple bolus function is generally unable to model a second passage of the contrast agent following a first passage thereof (for example, due to its re-circulation through the body-part, following the normal cycle of circulation of the blood in the patient). Particularly, when the second passage of the contrast agent reaches the body-part before completion of the first passage, the resulting time-intensity function is not very accurate in describing the actual trend of the echo signal over time and therefore it is unable to accurately describe the perfusion of the body-part by the contrast agent - with corresponding errors in the resulting perfusion parameter values, which adversely affect a quality of the analysis process.

In order to tackle this problem, several methods for separating the first passage from the second passage of the contrast agent have been proposed in the art - for example, as described in the above-mentioned documents US-B-6,216,094 and "R.A.F. Linton, N.W.F. Linton and D.M. Band". However, these methods become unreliable when there is a substantial degree of encroachment of the second passage on the first passage and/or when the second passage starts before a peak instant of the first passage.

Alternatively, the above-mentioned document WO-A-2006/067201 proposes the use of a combined bolus function that consists of the sum of a first simple bolus function (for the first passage of the contrast agent) and a second simple bolus function (for the second passage of the contrast agent). The combined bolus function allows obtaining an accurate representation of the first passage of the contrast agent, which contains the most relevant information about the perfusion of the corresponding location of the body-part (with the perfusion parameter values being calculated from the first time-intensity function facilitating its characterization).

However, the combined bolus function now includes a high number of fitting parameters (i.e., twice the ones of the simple bolus function). Therefore, the fitting of the echo signals by the combined bolus function (for determining the corresponding time-intensity functions) is quite problematic. Particularly, this may cause instabilities in the applied algorithm (for example, because of ambiguities or convergence errors), and/or it may result in unreliable estimates of the fitting parameters (and then of the corresponding perfusion parameters); the problem is especially important when the fitting is applied on noisy echo signals.

Summary

In its general terms, a solution according to one or more embodiments of the present invention is based on the idea of suitably initializing the fitting parameters.

Particularly, one or more aspects of the invention are set out in the independent claims, with advantageous features thereof that are set out in the dependent claims.

Brief description of the drawings

A solution according to one or more embodiments of the invention, as well as further features and the advantages thereof, will be best understood with reference to the following detailed description, given purely by way of a non-restrictive indication, to be read in conjunction with the accompanying drawings (wherein corresponding elements are denoted with equal or similar references and their explanation is not repeated for the sake of brevity, and the name of each entity is generally used to denote both its type and its attributes - such as its value, content and representation - for the sake of simplicity). Particularly:

FIG.1 shows a pictorial representation of an ultrasound scanner that can be used to practice the solution according to an embodiment of the invention,
FIG.2A shows an example of an echo signal representing the echo power recorded over time for a generic location of a body-part during an analysis process thereof,
FIG.2B shows an example of a time-intensity function based on a simple bolus function that fits this echo signal,
FIG.2C shows the echo signal and the time-intensity function of FIG.2B plotted on a semi-logarithmic scale,
FIG.2D shows an example of a time-intensity function based on a combined bolus function that fits the same echo signal,
FIG.2E shows the echo signal and the time-intensity function of FIG.2D plotted on a semi-logarithmic scale,
FIG.3A-FIG.3B shows an exemplary implementation of the solution according to an embodiment of the invention,
FIG.4A-FIG.4C, FIG.5A-FIG.5C, and FIG.6A-FIG.6C show different examples of application of the solution according to an embodiment of the invention, and
FIG.7 shows a collaboration diagram representing the roles of the main components that may be used to implement the solution according to an embodiment of the invention.

Detailed description

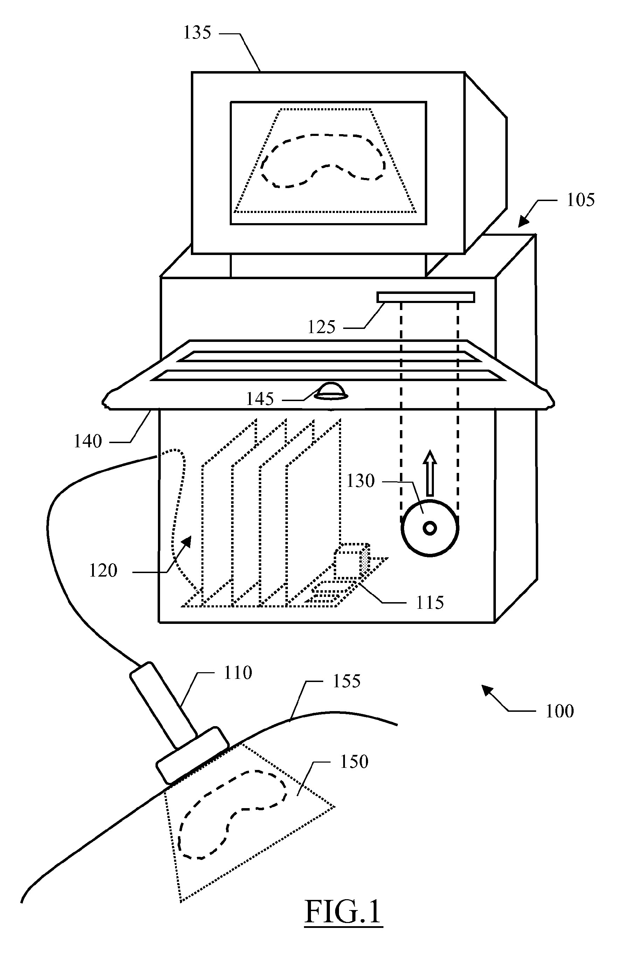

With reference in particular to Figure 1, an ultrasound scanner 100 is illustrated that can be used to practice the solution according to an embodiment of the invention. The ultrasound scanner 100 includes a central unit 105 and a hand-held transmit-receive imaging probe 110 (for example, of the array type). The imaging probe 110 transmits ultrasound waves consisting of a sequence of pulses (for example, having a center frequency between 1 and 50 MHz), and receives radio-frequency (RF) echo signals resulting from the reflection of the ultrasound pulses in a selected scanning plane; for this purpose, the imaging probe 110 is provided with a transmit/receive multiplexer, which allows using the imaging probe 110 in the above-described pulse-echo mode.

The central unit 105 houses a motherboard 115, on which the electronic circuits controlling operation of the ultrasound scanner 100 are mounted (for example, a microprocessor, a working memory and a hard-disk drive). Moreover, one or more daughter boards (denoted as a whole with the reference 120) are plugged into the motherboard 115; the daughter boards 120 provide the electronic circuits for driving the imaging probe 110 and for processing the received echo signals. The ultrasound scanner 100 can also be equipped with a drive 125 for removable disks 120 (such as CDs or DVDs). A monitor 125 displays images relating to an analysis process that is in progress. Operation of the ultrasound scanner 100 is controlled by means of a keyboard 140, which is connected to the central unit 105 in a conventional manner; preferably, the keyboard 140 is provided with a trackball 145 that is used to manipulate the position of a pointer (not shown in the figure) on a screen of the monitor 125.

The ultrasound scanner 100 is used to analyze a body-part 150 of a patient 155, in order to assess a corresponding blood perfusion. For this purpose, during an analysis process of the body-part 150 a contrast agent (acting as an efficient ultrasound reflector) is administered to the patient 155. For example, the contrast agent consists of a suspension of gas bubbles in a liquid carrier; typically, the gas bubbles have diameters of the order of 0.1-5 $\mu$m, so as to allow them to pass through the capillaries of the patient 155. The gas bubbles are generally stabilized by entraining or encapsulating the gas or a precursor thereof into a variety of systems, including emulsifiers, oils, thickeners, sugars, proteins or polymers; stabilized gas bubbles are generally referred to as gas-filled microvesicles. The microvesicles include gas bubbles dispersed in an aqueous medium and bound at the gas/liquid interface by a very thin envelope involving a surfactant (i.e., an amphiphilic material), also known as microbubbles. Alternatively, the microvesicles include gas bubbles that are surrounded by a solid material envelope formed of lipids or (natural or synthetic) polymers, also known as microballoons or microcapsules. Another kind of contrast agent includes a suspension of porous microparticles of polymers or other solids, which carry gas bubbles entrapped within the pores of the microparticles. Examples of suitable aqueous suspensions of microvesicles, in particular microbubbles and microballoons, and of the preparation thereof are described in EP-A-0458745, WO-A-91/15244, EP-A-0554213, WO-A-94/09829 and WO-A-95/16467. An example of a commercial contrast agent comprising gas-filled microvesicles is SonoVue® by Bracco International BV.

The contrast agent is administered to the patient 155 intravenously as a bolus - i.e., a single dose provided by hand with a syringe over a short period of time (of the order of 2-20 seconds). The contrast agent circulates within a vascular system of the patient 155, so as to perfuse the body-part 150. At the same time, the imaging probe 110 is placed in contact with the skin of the patient 155 in the area of the body-part 150. A series of ultrasound pulses with low acoustic energy (such as with a mechanical index MI=0.01-0.1) is applied to the body-part 150, so as to induce a negligible destruction of the contrast agent (such as less than 5%, and preferably less than 1% of its local concentration between successive ultrasound pulses). The echo signals that are recorded in response to the ultrasound pulses - at successive acquisition instants over time during an analysis interval (for example, at a rate of 10-20 acquisitions per second during 1-3 minutes) - provide a representation of basic portions of the body-part 150 (in a slice thereof corresponding to the selected scanning plane) during the analysis process. The echo signals result from the superimposition of different contributions generated by the contrast agent (if present) and the surrounding tissue. Preferably, the ultrasound scanner 100 operates in a contrast-specific imaging mode so as to substantially remove, or at least reduce, the dominant (linear) contribution of tissue in the echo signals, with respect to the (non-linear) contribution of the contrast agent; examples of contrast-specific imaging modes include harmonic imaging (HI), pulse inversion (PI), power modulation (PM) and contrast pulse sequencing (CPS) techniques, as described, for example, in "Rafter et al., Imaging technologies and techniques, Cardiology Clinics 22 (2004), pp. 181-197".

The echo signal of each location of the body-part 150 (consisting of one or more basic portions thereof) is then fitted by an instance of a (model) parametric function of time, referred to as time-intensity function; particularly, the time-intensity function is defined by the values of a set of fitting parameters of the parametric function; these fitting parameter values are chosen as those that make the resulting time-intensity function best follow a trend of the echo signal over time. The time-intensity functions so obtained can then be used to calculate the values of different perfusion parameters of the corresponding locations of the body-part 150 (providing useful information for their characterization); examples of these perfusion parameters are a Time to Peak, or *TP* (representing a time required to reach a maximum of the echo signal), a mean Transit Time, or *mTT* (representing a time required by the contrast agent to perfuse the body-part), and an Area Under the Curve, or *AUC* (representing a relative regional tissue blood volume).

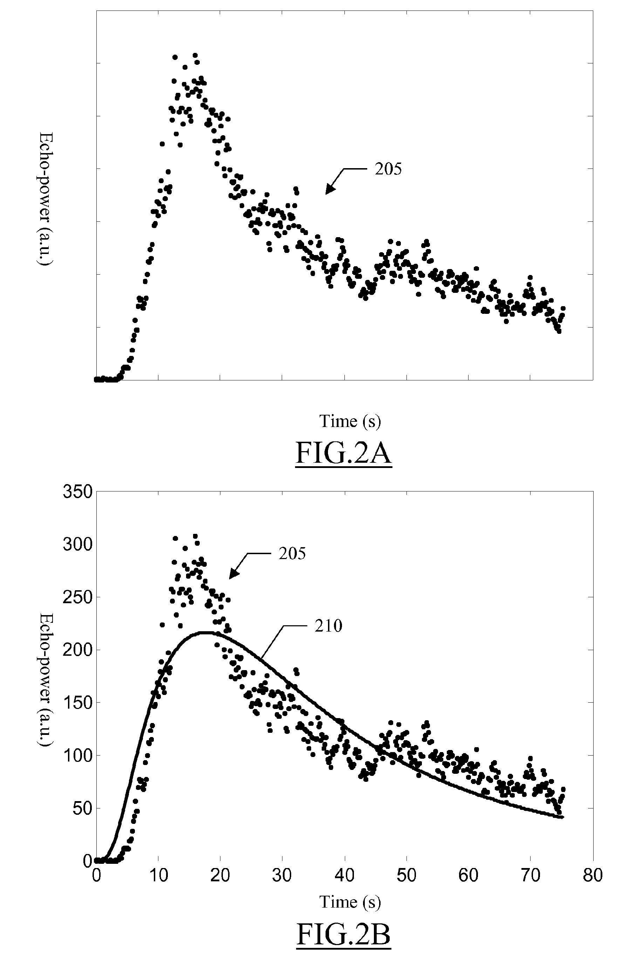

An example of an echo signal representing the echo power recorded over time for a generic location of a body-part during the analysis process is shown in FIG.2A. Particularly, the figure includes a diagram with a sequence of signal dots 205, which represent the echo power - on the ordinate axis in terms of arbitrary units (a.u.) - recorded at the time of the corresponding acquisition instants - on the abscissa axis in seconds from the time of administration of the contrast agent. The echo signal 205 was obtained *in vivo* by imaging a prostate after a bolus injection of 2.4 mL of SonoVue.

The contrast agent administered to the patient circulates within his/her vascular system so as to perfuse the body-part under analysis. Particularly, during a first (main) passage of the contrast agent through the body-part following its administration (from 0s to 15s in the example at issue), the echo power increases as a result of a wash-in of the contrast agent that reaches the body-part; once the echo power has reached its peak (at about 15s in the example at issue), it starts decreasing as a result of a wash-out of the contrast agent that leaves the body-part (from 15s to 75s in the example at issue).

In the meanwhile, the contrast agent continues to circulate through the vascular system of the patient during the wash-out phase in the body part, being gradually diluted in the blood so that a new inflow of the contrast agent in the body-part decreases at every next (secondary) passage thereof - substantially disappearing, for example, after a second passage. Therefore, during the wash-out phase the echo signal 205 exhibits a transient increase (lower than the above-mentioned peak) as a result of the second passage of the contrast agent (from 20s to 75s in the example at issue).

An example of a time-intensity function (based on a simple bolus function) that fits the echo signal 205 of FIG.2A is shown in FIG.2B. This time-intensity function is represented with a curve 210, which plots the echo power (on the ordinate axis) as a function of time (on the abscissa axis).

Particularly, the time-intensity function 210 is an instance of a simple bolus function that consists of the lognormal distribution function (wherein the natural logarithm of its independent variable follows a normal distribution function):

$$B_s(t) = O_s + A_s \cdot \frac{e^{-\frac{[\ln(t)-m_s]^2}{2s_s^2}}}{t \cdot s_s \cdot \sqrt{2\pi}},$$

where $t$ is the independent variable representing the time (measured from the time of administration of the contrast agent) and $B_s(t)$ is the dependent variable representing the echo power (at the time $t$); the simple bolus function $B_s(t)$ includes a fitting parameter $O_s$ (an offset), a fitting parameter $A_s$ (an amplitude factor representing the perfusion parameter $AUC$), a fitting parameter $m_s$ (the mean of the corresponding distribution of the natural logarithm of the variable $t$), and a fitting parameter $s_s$ (the standard deviation of the corresponding distribution of the natural logarithm of the variable $t$). The specific instance of the simple bolus function $B_s(t)$ defining the time-intensity function 210 is then determined by the actual values of its fitting parameters $O_s$, $A_s$, $m_s$ and $s_s$.

As can be seen, the time-intensity function 210 roughly follows the typical trend of the echo signal 205 over time (with its wash-in phase, peak, and wash-out phase). However, the resulting fitting is not very accurate, so that the time-intensity function 210 may provide erroneous values of the resulting perfusion parameters. For example, the values of the perfusion parameters $AUC$ and $mTT$ being calculated from the time-intensity function 210 are:

$$AUC = A_s = 10,347,$$

and

$$mTT = e^{m_s + \frac{s_s^2}{2}} = 45.14\,s.$$

The values of the same perfusion parameters $AUC$ and $mTT$ being calculated directly (i.e., numerically) from a portion of the echo signal 205 including its peak (for example, from 0s to 25s) are instead:

$$AUC = 3,693,$$

and

$$mTT = 16.26\,s.$$

Therefore, the values of the perfusion parameters *AUC* and *mTT* derived from the time-intensity function 210 are largely overestimated (as compared to their actual values calculated from the echo signal 205).

The discrepancy between the values of the perfusion parameters derived from the time-intensity function 210 and calculated from the echo signal 205 is mainly due to limitations of the simple bolus function $B_s(t)$ in modeling the second passage of the contrast agent through the body-part; indeed, the simple bolus function $B_s(t)$ only describes bolus kinetics of perfect mixing chambers, as used in indicator dilution theory (without any re-circulation). Particularly, the simple bolus function $B_s(t)$ is characterized by a single exponential decay rate during the wash-out phase; conversely, the second passage of the contrast agent causes a change in the exponential decay rate of the echo signal 205 during the wash-out phase, which cannot be modeled by the simple bolus function $B_s(t)$.

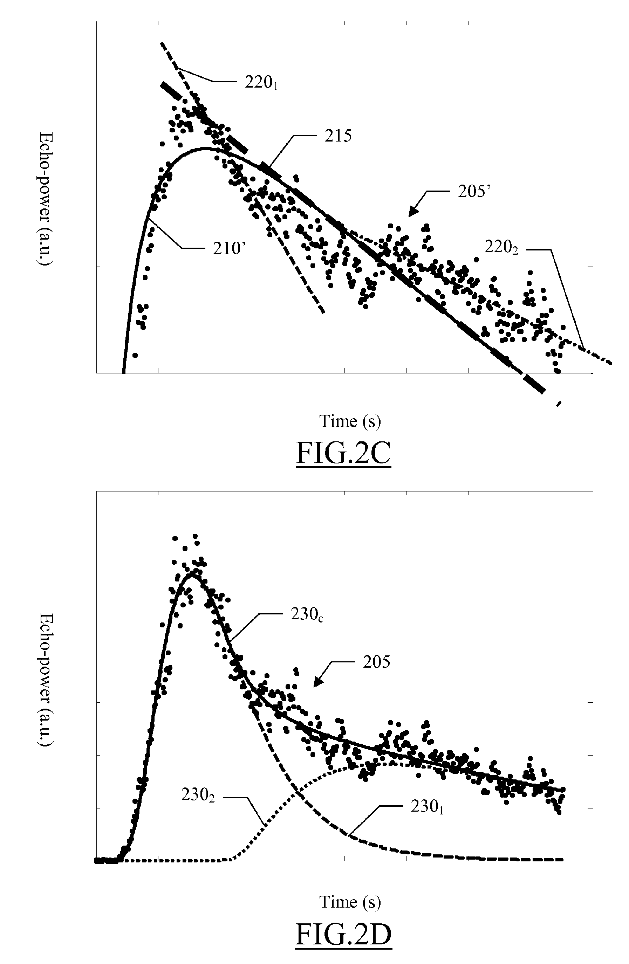

This is better explained in FIG.2C, which shows the echo signal and the time-intensity function of FIG.2B plotted on a semi-logarithmic scale (on the ordinate axis for the echo power). In this case, the echo signal and the time-intensity function are represented with a sequence of signal dots 205' and a time-intensity curve 210', respectively (which provide the logarithm of the echo power as a function of time).

Therefore, on a logarithmic scale, the exponential decay rates of the echo signal 205' and of the time-intensity function 210' are now represented by their slopes. As can been seen, the time-intensity function 210' has a constant exponential decay rate, as indicated by a straight line 215 being tangent to its curve (in thick dashed line). On the contrary, the echo signal 205' has two different exponential decay rates. Particularly, at the beginning of the wash-out phase, the echo signal 205' has a higher exponential decay rate, due to the wash-out of the first (main) passage of the contrast agent, as indicated by a straight line $220_1$ (in thin dashed line); at a later instant, the echo signal 205' has a lower exponential decay rate, due to the wash-out of the second passage of the contrast agent, as indicated by a straight line $220_2$ (in thin dashed line).

An example of a time-intensity function (based on a combined bolus function) that fits the same echo signal 205 of FIG.2A is instead shown in FIG.2D. This time-intensity function is represented with a curve $230_c$ (again plotting the echo power as a function of time).

Particularly, the time-intensity function $230_c$ is an instance of a combined bolus function that consists of the sum of a first (simple) bolus function for the first passage of the contrast agent and a second (simple) bolus function for the second passage of the contrast agent:

$$B_c(t) = O_c + A_1 \cdot \frac{e^{-\frac{[\ln(t)-m_1]^2}{2s_1^2}}}{t \cdot s_1 \cdot \sqrt{2\pi}} + A_2 \cdot \frac{e^{-\frac{[\ln(t-\Delta t)-m_2]^2}{2s_2^2}}}{t \cdot s_2 \cdot \sqrt{2\pi}} \ ;$$

the combined bolus function $B_c(t)$ now includes a fitting parameter $O_c$ (a common offset), a fitting parameter $\Delta t$ (representing a time delay of the second passage with respect to the first passage), the fitting parameters $A_1$, $m_1$, $s_1$ as above referring to the first passage of the contrast agent, and the fitting parameters $A_2$, $m_2$, $s_2$ as above referring to the second passage of the contrast agent. The specific instance of the combined bolus function $B_c(t)$ defining the time-intensity function $230_c$ is then determined by the actual values of its fitting parameters $O_c$, $A_1$, $m_1$, $s_1$, $A_2$, $m_2$, $s_2$ and $\Delta t$.

In addition, it is also possible to determine (from the time-intensity function $230_c$) the instance of the first bolus function $B_1(t)$ (referred to as first time-intensity function) and the instance of the second bolus function $B_2(t)$ (referred to as second time-intensity function), which are represented in the same diagram with a curve $230_1$ and a curve $230_2$, respectively. Therefore, the first time-intensity function $230_1$ represents the evolution over time of the echo power during the first passage of the contrast agent, whereas the second time-intensity function $230_2$ represents the evolution over time of the echo power during the second passage of the contrast agent. As a result, it is possible to separate the first passage of the contrast agent (containing the most relevant information about the perfusion of the corresponding location of the body-part) from its second passage.

In this case, the time-intensity function $230_c$ precisely follows the trend of the echo signal 205 (with its wash-in phase, peak, and wash-out phase). Moreover, the resulting fitting is very accurate, so that the time-intensity function $230_c$ provides correct values of the resulting perfusion parameters. For example, the values of the perfusion parameters *AUC* and *mTT* being derived from the first time-intensity function $230_1$:

$$AUC = A_1 = 5,071,$$

and

$$mTT = e^{m_1 + \frac{s_1^2}{2}} = 20.71s$$

are very similar to their actual values calculated from the echo signal 205 - i.e., *3,693* and *16.26s,* respectively.

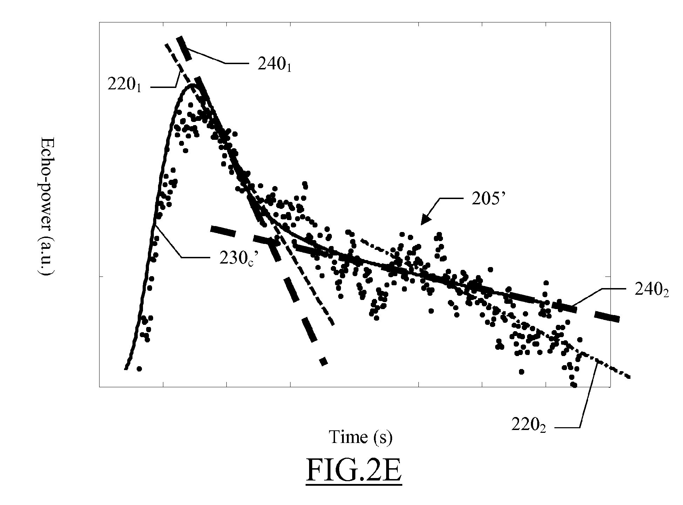

Indeed, the combined bolus function $B_c(t)$ is now capable of accurately modeling the first and second passages of the contrast agent through the body-part thanks to its double exponential decay rate during the wash-out phase. This is better explained in FIG.2E, which shows the echo signal and the time-intensity function of FIG.2D plotted on a semi-logarithmic scale. In this case, the echo signal is represented with the same sequence of signal dots 205' as above, while the time-intensity function is represented with a curve $230_c$' (again displaying the logarithm of the echo power as a function of time). As can been seen, the time-intensity function $230_c$' now has two different exponential decay rates (like the echo signal 205'). Particularly, at the beginning of the wash-out phase the time-intensity function $230_c$' has a higher exponential decay rate, as indicated by a straight line $240_1$ being tangent to its curve (in thick dashed line); at a later instant, the time-intensity function $230_c$' has a lower exponential decay rate, as indicated by a straight line $240_2$ being tangent to its curve (in thick dashed line). These exponential decay rates $240_1$ and $240_2$ then correspond to the different exponential decay rates $220_1$ and $220_2$ (in thin dashed line) of the echo signal 205' - being due to the wash-out during the first passage and the second passage of the contrast agent, respectively.

However, the combined bolus function $B_c(t)$ includes a high number of fitting parameters $O_c$, $A_1$, $m_1$, $s_1$, $A_2$, $m_2$, $s_2$ and $\Delta t$; therefore, the fitting of each echo signal by the combined bolus function $B_c(t)$ for determining the corresponding time-intensity function requires the optimization of at least seven fitting parameters - disregarding the fitting parameter $O_c$ that may be assessed separately.

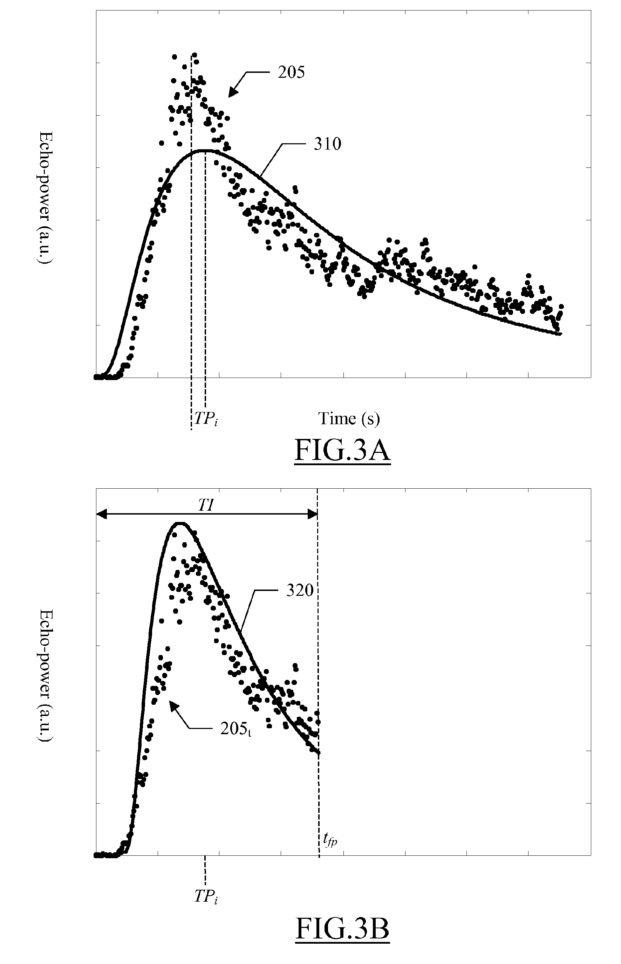

For this purpose, in a solution according to an embodiment of the invention a dedicated procedure for initializing the fitting parameters $A_1$, $m_1$, $s_1$, $A_2$, $m_2$, $s_2$ and $\Delta t$ is provided. Particularly, an exemplary implementation of this solution is shown in FIG.3A-FIG.3B.

Starting from FIG.3A, the echo signal 205 is at first fitted by an instance of the simple bolus function $B_s(t)$ - referred to as initial time-intensity function (defined by corresponding values of its fitting parameters, referred to as initial fitting parameter values $O_i$, $A_i$, $m_i$ and $s_i$); the initial time-intensity function is represented in the same diagram of FIG.2A with a curve 310. The initial time-intensity function 310 is then used to calculate the value of its time to peak *TP* (referred to as initial time-to-peak value $TP_i$).

As pointed out above, the accuracy of the initial time-intensity function 310 is not very high, so that the initial time-to-peak value $TP_i$ only provides a rough estimate of its actual peak instant $TP_i$' in the echo signal 205 (for example, $TP_i=17.5s$ against $TP_i'=15s$ in the example at issue). However, this is not a problem, since the above-described operation is only aimed at identifying a portion of the echo signal 205 for the initialization of the fitting parameters of the combined bolus function $B_c(t)$ (for which no particular precision is required).

Moving to FIG.3B, a first passage time $t_{fp}$ is calculated from the initial time-to-peak value $TP_i$ (for example, equal to twice the time-to-peak value $TP_i$ - i.e., $t_{fp}=2\cdot17.5=35s$ in the example at issue). The first passage time $t_{fp}$ defines a truncation interval *TI* (from the time of administration of the contrast agent to the first passage time $t_{fp}$). A truncated echo signal (represented with a corresponding sequence of signal dots $205_t$) is then obtained by discarding the echo signal outside the truncation interval *TI*. Therefore, to a first approximation, the truncated echo signal $205_t$ may be deemed representative of the first passage of the contrast agent only (since in this phase the contribution of the second passage of the contrast agent is limited).

The truncated echo signal $205_t$ is now fitted by another instance of the same simple bolus function $B_s(t)$ - referred to as truncated time-intensity function (again defined by corresponding values of its fitting parameters - referred to as truncated fitting parameter values $O_t$, $A_t$ $m_t$ and $s_t$); the truncated time-intensity function is represented in the same diagram with a curve 320. Therefore, the truncated time-intensity function 320 now follows the trend of the truncated echo signal $205_t$ over time with a higher accuracy (because of the reduced contribution of the second passage of the contrast agent in the truncated interval *TI*). The truncated fitting parameter values $A_t$, $m_t$, and $s_t$ are then used to initialize the fitting parameters $A_1$, $m_1$, $s_1$, $A_2$, $m_2$, $s_2$ and $\Delta t$ of the combined bolus function $B_c(t)$ for fitting the original (complete) echo signal (for example, by setting them to a predefined multiple thereof).

The above-described solution according to an embodiment of the invention strongly facilitates the fitting operations (of the echo signals by the combined bolus function $B_c(t)$). As a result, it is possible to avoid (or at least substantially reduce) any risk of instabilities in the applied algorithm. In any case, this increases the accuracy of the time-intensity functions (in precisely describing the perfusion of the body-part by the contrast agent), providing a more robust estimate of the perfusion parameters that are calculated from the time-intensity functions. All of the above has a beneficial effect on the quality of the analysis process. These advantages are particularly evident when the fitting is applied on noisy echo signals.

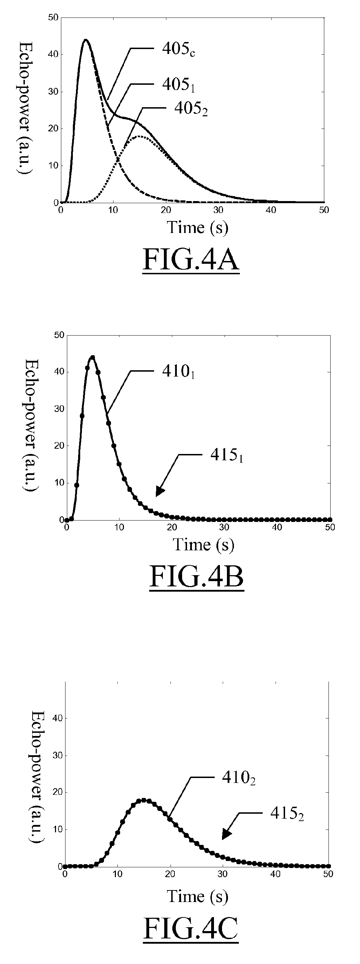

An example of application of the solution according to an embodiment of the invention is shown in FIG.4A-FIG.4C.

Particularly, starting from FIG.4A a first test function was generated to simulate a first passage of the contrast agent through the body-part; the first test function is represented in the figure with a corresponding curve $405_1$ (plotted as a dashed line); likewise, a second test function was generated to simulate a second passage of the contrast agent; the second time-intensity function is represented in the figure with a corresponding curve $405_2$ (plotted as a dotted line). A combined test function was calculated by summing the first test function and the second test function; the combined test function is represented in the figure with a corresponding curve $405_c$ (plotted as a solid line). A test data array was then obtained by evaluating the combined test function $405_c$ over time. In this case, the second test function $405_2$ has a substantial degree of encroachment on the first test function $405_1$, resulting in a highly distorted combined test function $405_c$.

The fitting of this test data array with the application of the above-described solution provided a first time-intensity function that is represented in FIG.4B with a corresponding curve $410_1$. A first test data array (obtained by evaluating the first test function over time) is represented in the figure with a corresponding sequence of test dots $415_1$. As can be seen, the first time-intensity function $410_1$ superimposes perfectly on the first test data array $415_1$.

Likewise, the same fitting provided a second time-intensity function that is represented in FIG.4C with a corresponding curve $410_2$. A second test data array (obtained by evaluating the second test function over time) is represented in the figure with a corresponding sequence of test dots $415_2$. As can be seen, the second time-intensity function $410_2$ superimposes perfectly on the second test data array $415_2$.

Therefore, the proposed solution is able to represent the first and second passages perfectly, even when there is a substantial degree of encroachment of the second passage on the first passage. This is confirmed by the values of the fitting parameters of the first and second time-intensity functions (i.e., *mTT=7, s=0.5* and *AUC=300* for the first time-intensity function, and *mTT=18, s=0.35* and *AUC=250* for the second time-intensity function), which are exactly the same as the values of the corresponding fitting parameters used for generating the first and second test functions.

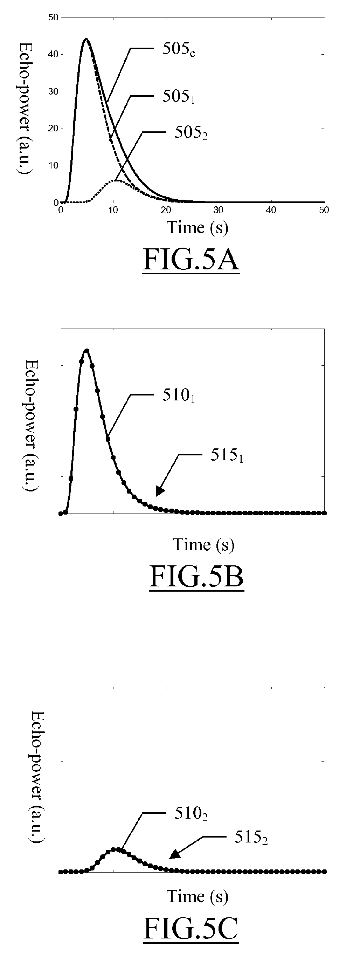

A further example of application of the solution according to an embodiment of the invention is shown in FIG.5A-FIG.5C.

Particularly, starting from FIG.5A a first test function (represented in the figure with a corresponding curve $505_1$ plotted as a dashed line) and a second test function (represented in the figure with a corresponding curve $505_2$ plotted as a dotted line) were generated as above to simulate a first passage and a second passage of the contrast agent through the body-part, respectively; a combined test function (represented in the figure with a corresponding curve $505_c$ plotted as a solid line) was calculated by summing the first test function and the second test function, and a test data array was then obtained by evaluating the combined test function $505_c$ over time. In this case, the second test function $505_2$ is totally encroached with the first test function $505_1$ (since the second test function $505_2$ starts before the peak instant of the first test function $505_1$), resulting in a minimal distortion of the combined test function $505_c$.

The fitting of this sequence of test data with the application of the above-described solution provided a first time-intensity function that is represented in FIG.5B with a corresponding curve $510_1$. A first test data array (obtained by evaluating the first test function over time) is represented in the figure with a corresponding sequence of test dots $515_1$. As can be seen, the first time-intensity function $510_1$ superimposes perfectly on the first test data array $515_1$.

Likewise, the same fitting provided a second time-intensity function that is represented in FIG.5C with a corresponding curve $510_2$. A second test data array (obtained by evaluating the second test function over time) is represented in the figure with a corresponding sequence of test dots $515_2$. As can be seen, the second time-intensity function $510_2$ superimposes perfectly on the second test data array $515_2$.

Therefore, the proposed solution is able to represent the first and second passages perfectly, even when there is a total encroachment of the second passage on the first passage. This is confirmed by the values of the fitting parameters of the first and second time-intensity functions (i.e., *mTT=7, s=0.5* and *AUC=300* for the first time-intensity function, and *mTT=12, s=0.3* and *AUC=50* for the second time-intensity function), which are exactly the same as the values of the corresponding fitting parameters used for generating the first and second test functions.

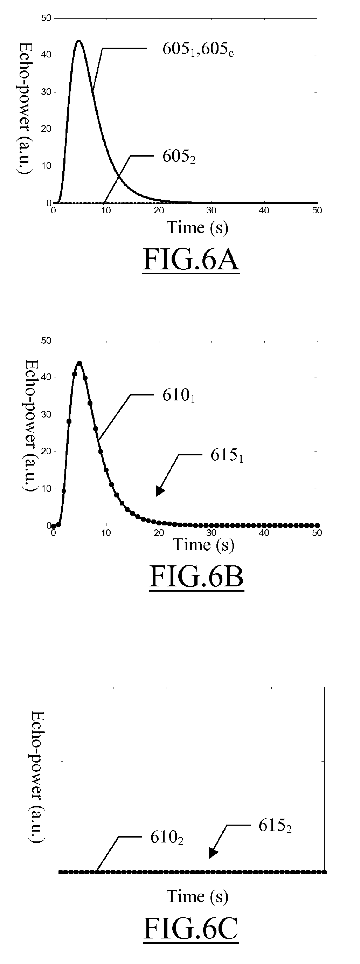

A different example of application of the solution according to an embodiment of the invention is shown in FIG.6A-FIG.6C.

Particularly, starting from FIG.6A a first test function (represented in the figure with a corresponding curve $605_1$ plotted as a dashed line) and a second test function (represented in the figure with a corresponding curve $605_2$ plotted as a dotted line) were generated as above to simulate a first passage and a second passage of the contrast agent through the body-part, respectively; a combined test function (represented in the figure with a corresponding curve $605_c$ plotted as a solid line) was calculated by summing the first test function and the second test function, and a test data array was then obtained by evaluating the combined test function $605_c$ over time. In this case, the second test function $605_2$ always remains zero (i.e., no re-circulation is present), with the combined test function $605_c$ that is completely determined by the first test function $605_1$.

The fitting of this sequence of test data with the application of the above-described solution provided a first time-intensity function that is represented in FIG.6B with a corresponding curve $610_1$. A first test data array (obtained by evaluating the first test function over time) is represented in the figure with a corresponding sequence of test dots $615_1$. As can be seen, the first time-intensity function $610_1$ superimposes perfectly on the first test data array $615_1$.

Likewise, the same fitting provided a second time-intensity function that is represented in FIG.6C with a corresponding curve $610_2$. A second test data array (obtained by evaluating the second test function over time) is represented in the figure with a corresponding sequence of test dots $615_2$. As can be seen, the second time-intensity function $610_2$ superimposes perfectly on the second test data array $615_2$.

Therefore, the proposed solution is able to represent the first and second passages perfectly, even in the extreme situation when the second passage of the contrast agent is absent. This is confirmed by the values of the fitting parameters of the first and second time-intensity functions (i.e., *mTT=7, s=0.5* and *AUC=300* for the first time-intensity function, and *AUC<0.001* for the second time-intensity function), which are substantially the same as the values of the corresponding fitting parameters used for generating the first and second test functions (i.e., *mTT=7, s=0.5* and *AUC=300* for the first test function, and *AUC=0* for the second test function).

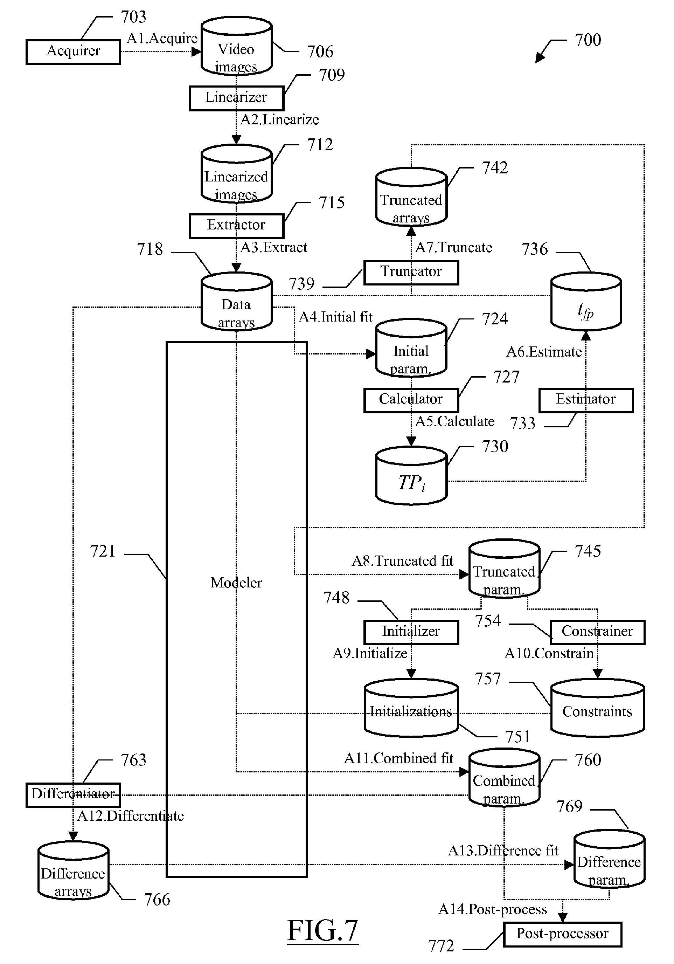

A collaboration diagram representing the roles of the main software and/or hardware components that may be used to implement the solution according to an embodiment of the invention is illustrated in FIG.7. These components are denoted as a whole with the reference 700; particularly, the information (programs and data) is typically stored on the hard-disk and loaded (at least partially) into the working memory of the ultrasound scanner when the programs are running, together with an operating system and other application programs (not shown in the figure). The programs are initially installed onto the hard disk, for example, from DVD-ROM. More specifically, the figure describes the static structure of the system (by means of the corresponding components) and its dynamic behavior (by means of a series of exchanged messages, each one representing a corresponding action, denoted with sequence numbers preceded by the symbol "A").

Particularly, an acquirer 703 includes a driver that controls the imaging probe. For example, this driver is provided with a transmit beam former and pulsers for generating the ultrasound pulses to be applied to the body-part under analysis; the imaging probe then receives the analog RF echo signals that are reflected by the different basic portions of the body-part in its slice at the selected scanning plane. These analog RF echo signals are supplied to a receive processor, which pre-amplifies the analog RF echo signals and applies a preliminary time-gain compensation (TGC); the analog RF echo signals are then converted into digital values by an Analog-to-Digital Converter (ADC), and combined into focused beam signals through a receive beam former. The digital echo signals so obtained are preferably processed through further digital algorithms and other linear or non-linear signal conditioners (for example, a post-beam-forming TGC). Particularly, the receive processor applies a contrast-specific algorithm to suppress the contribution of the tissue (such as based on the above-mentioned HI, PI, PM or CPS techniques). The digital echo signals are then demodulated, log-compressed (in order to obtain images with well-balanced contrast), and scan-converted into a video format. This process generates a sequence of contrast-specific video images, each one representing the selected slice of the body-part at the corresponding acquisition instant. Each video image is defined by a matrix of cells (for example, with 512 rows x 512 columns) for pixels representing the different basic portions of the body-part. Each cell of the video image stores a pixel value (for example, coded on 8 bits) that defines a brightness of the corresponding pixel; for example, in grayscale video images the pixel value increases from 0 (black) to 255 (white) as a function of the echo signal intensity of the corresponding basic portion of the body-part.

At the beginning of the analysis process, an operator of the ultrasound scanner actuates the imaging probe and moves it around the body-part to be analyzed (before administering any contrast agent). The corresponding video images are displayed in real-time as soon as they are acquired; the operator then chooses a slice of the body-part to be analyzed (preferably including a suspicious lesion) and keeps the imaging probe in a fixed position. The contrast agent is now administered to the patient, and the ultrasound scanner acquires a sequence of video images that represents this slice of the body-part over time; the sequence of video images so obtained is saved into a repository 706 for off-line analysis (action "A1.Acquire").

The video images 706 are supplied to a linearizer 709, which processes each pixel value thereof so as to make it directly proportional to the corresponding local concentration of the contrast agent. For example, this result can be achieved by applying an inverse log-compression (to reverse the effect of its application by the acquirer 703), and then squaring the values so obtained (as described in WO-A-2004/110279). Typically, this operation is limited to a portion of the video images 706, corresponding to a region of interest of the body-part that has been selected by the operator (for example, by drawing a line around it on one of the video images 706 with the help of the trackball). As a result, the linearizer 709 generates a corresponding sequence of linearized images, which is saved into a repository 712 (action "A2.Linearize").

An extractor 715 accesses the repository 712 of the linearized images for extracting one or more data arrays each one for a corresponding location of the body-part. Particularly, when the analysis process is performed at the level of pixels, a data array is created for each pixel of the linearized images 712; the data array includes a sequence of the corresponding pixel values along the linearized images 712. Conversely, when the analysis process is performed at the level of groups of pixels, in each linearized image 712 a single group value is calculated for each group of pixels (for example, by averaging the corresponding pixel values); a data array is then created for each group of pixels by including a sequence of the corresponding group values along the linearized images 712. Moreover, the analysis process may also be performed at the level of the whole region of interest, wherein a single data array is created from the corresponding pixel values as described above. In any case, the data arrays are then stored into a repository 718 (action "A3.Extract").

The data arrays 718 are supplied to a modeler 721. The modeler 721 at first fits each data array 718 by an instance of the simple bolus function $B_s(t)$, so as to obtain the corresponding initial time-intensity function (being defined by the initial fitting parameter values $O_i$, $A_i$, $m_i$ and $s_i$). This result is achieved by applying well known error-minimization algorithms; for example, when the fitting operation is based on the gradient descent algorithm, at each iteration of an optimization loop a direction is determined along which a difference between a current instance of the simple bolus function $B_s(t)$ and the data array 718 decreases most rapidly (i.e., the corresponding gradient is the lowest); the fitting parameters of the simple bolus function $B_s(t)$ are then updated in this direction for a next iteration of the optimization loop (until the difference is smaller than a pre-defined threshold value). The sets of initial fitting parameter values $O_i$, $A_i$, $m_i$ and $s_i$ for the different locations (i.e., pixels, groups of pixel, or region of interest) are stored into a repository 724 (action "A4.Initial fit").

The sets of initial fitting parameter values $O_i$, $A_i$, $m_i$ and $s_i$ are supplied from the repository 724 to a calculator 727. For each location, the calculator 727 calculates the initial time-to-peak value $TP_i$ of the corresponding initial time-intensity function:

$$TP_i = e^{m_i - s_i^2}.$$

The time-to-peak values $TP_i$ for the different locations are stored into a repository 730 (action "A5.Calculate"). This feature is particularly advantageous, since it allows calculating the peak instant of the echo signal in each location analytically.

The time-to-peak values $TP_i$ are then supplied from the repository 730 to an estimator 733. For each location, the estimator 733 calculates the corresponding first passage time $t_{fp}$. Particularly, the first passage time $t_{fp}$ is set to:

$$t_{fp} = \beta \cdot TP_i,$$

wherein $\beta$ is a factor, for example, comprised between 1 and 3, and preferably between 1.5 and 2.5 (such as $\beta=2$). The first passage times $t_{fp}$ for the different locations are stored into a repository 736 (action "A6.Estimate").

A truncator 739 accesses both the repository 718 of the data arrays and the repository 736 of the first passage times $t_{fp}$. For each location, the truncator 739 truncates the corresponding data array 718 by discarding the values thereof outside the truncation interval TI, which is defined from the time origin (corresponding to the time of administration of the contrast agent) to the first passage time $t_{fp}$. For this purpose, a first passage number $N_{fp}$ is calculated as the integer of the ratio between the first passage time $t_{fp}$ and an acquisition period of the video images 706; the data array 718 is then truncated by limiting it to the values thereof up to the first passage number $N_{fp}$. An indication of the truncated (data) arrays so obtained for the different locations are stored into a repository 742 (action "A7.Truncate"); for example, each truncated array is defined by its first passage number $N_{fp}$ and a link to the associated data array 718 (so as to avoid duplicating the corresponding data).

The truncated arrays 742 are again supplied to the modeler 721. The modeler 721 now fits each truncated array 742 by an instance of the same simple bolus function $B_s(t)$, so as to obtain the corresponding truncated time-intensity function (being defined by the truncated fitting parameter values $O_t$, $A_t$, $m_t$ and $s_t$). The sets of truncated fitting parameter values $O_t$, $A_t$, $m_t$ and $s_t$ for the different locations are stored into a repository 745 (action "A8.Truncated fit").

An inizializer 748 accesses the repository 745 of the truncated fitting parameter values $O_t$, $A_t$, $m_t$ and $s_t$. For each location, the inizializer 748 initializes the fitting parameters $A_1$, $m_1$, $s_1$, $A_2$, $m_2$, $s_2$ and $\Delta t$ of the combined bolus function $B_c(t)$ for its fitting on the corresponding data array 718 (the fitting parameter $O_c$ is not initialized since it is assessed separately). For this purpose, each fitting parameter $A_1,A_2$ and $s_1,s_2$ is set to a multiple of the corresponding truncated fitting parameter value $A_t$ and $s_t$, respectively; for example, the fitting parameters $A_1$, $s_1$, $A_2$ and $s_2$ are set to:

$$A_1 = A_t,$$

$$s_1 = s_t,$$

$$A_2 = 5 \cdot A_t,$$

and

$$s_2 = 1.1 \cdot s_t.$$

It should be noted that since the second bolus function $B_2(t)$ is completely independent of the first bolus function $B_1(t)$, the values of the fitting parameters $A_2$ and $s_2$ may significantly differ from the corresponding truncated fitting parameter values $A_t$ and $s_t$; therefore, the fitting parameters $A_2$ and $s_2$ are set to the corresponding truncated fitting parameter values $A_t$ and $s_t$ multiplied by a factor generally different from 1 (i.e., 5 and 1.1 in the example at issue).

Moreover, the inizializer 748 calculates the value of the mean transit time $mTT$ for the truncated time-intensity function (referred to as truncated mean transit time value $mTT_t$):

$$mTT_t = e^{m_t + \frac{s_t^2}{2}}.$$

The truncated mean transit time value $mTT_t$ is used to set the mean transit time $mTT$ for the first bolus function $B_1(t)$ and the second bolus function $B_2(t)$ of the combined bolus function $B_c(t)$ (denoted with $mTT_1$ and $mTT_2$, respectively) as above; for example, the mean transit times $mTT_1$ and $mTT_2$ are set to:

$$mTT_1 = mTT_t,$$

and

$$mTT_2 = 2 \cdot mTT_t.$$

The initialization of the fitting parameters $m_1$ and $m_2$ is then obtained by calculating them from the initialization values of the mean transit times $mTT_1$ and $mTT_2$, respectively (in addition to the initialization values of the fitting parameters $s_1$ and $s_2$, respectively):

$$m_1 = \log(mTT_1) - \frac{s_1^2}{2} = \log(mTT_t) - \frac{s_t^2}{2},$$

and

$$m_2 = \log(mTT_2) - \frac{s_2^2}{2} = \log(2 \cdot mTT_t) - \frac{(1.1 \cdot s_t)^2}{2}.$$

At the end, the fitting parameter $\Delta t$ as well is set to a multiple of the truncated mean transit time value $mTT_t$; for example, the fitting parameter $\Delta t$ is set to:

$$\Delta t=0.5 \cdot mTT_t.$$

The sets of initialized fitting parameters of the combined bolus function $B_c(t)$ so obtained for the different locations are stored into a repository 751 (action "A9.Initialize").

At the same time, a constrainer 754 accesses the same repository 745 of the truncated fitting parameter values $O_t$, $A_t$, $m_t$ and $s_t$. For each location, the constrainer 754 calculates a set of constraints for the combined bolus function $B_c(t)$ (for its fitting on the corresponding data array 718). For this purpose, each fitting parameter $A_1,A_2$ and $s_1,s_2$ is constrained to vary (during the fitting operation) within a value range being defined by a lower limit and an upper limit that are set to multiples of the corresponding truncated fitting parameter value $A_t$ and $s_t$, respectively; for example, the fitting parameters $A_1$, $s_1$, $A_2$ and $s_2$ are constrained to vary within the value ranges:

$$A_1=from\ 0.9{\cdot}A_t\ to\ 1.25{\cdot}A_t,$$

$$s_1=from\ 0.9{\cdot}s_t\ to\ 1.25{\cdot}s_t,$$

$$A_2=from\ 0\ to\ 20{\cdot}A_t,$$

and

$$s_2=from\ 0\ to\ 2{\cdot}s_t.$$

Likewise, the constrainer 754 calculates value ranges for each mean transit time $mTT_1$ and $mTT_2$, between a lower limit and an upper limit that are set to multiples of the truncated mean transit time value $mTT_t$; for example, the value ranges of the mean transit times $mTT_1$ and $mTT_2$ are set to:

$$mTT_1=from\ 0.9{\cdot}mTT_t\ to\ 1.25{\cdot}mTT_t,$$

and

$$mTT_2=from\ mTT_t\ to\ 5{\cdot}mTT_t.$$

The constraints of the fitting parameters $m_1$ and $m_2$ are obtained as above from the value ranges of the mean transit times $mTT_1$ and $mTT_2$, respectively (in addition to the value ranges of the fitting parameters $s_1$ and $s_2$, respectively):

$$m_1=from\ \log(0.9 \cdot mTT_t) - \frac{(0.9 \cdot s_t)^2}{2}\ to\ \log(1.25 \cdot mTT_t) - \frac{(1.25 \cdot s_t)^2}{2},$$

and

$$m_2=from\ m_2 = \log(mTT_t)\ to\ \log(5 \cdot mTT_t) - \frac{(2 \cdot s_t)^2}{2}.$$

Moreover, the fitting parameter $\Delta t$ as well is constrained to vary within a value range being defined by a lower limit and an upper limit that are set to multiples of the same truncated mean transit time value $mTT_t$; for example, the fitting

parameter $\Delta t$ is constrained to vary within the value range:

$$\Delta t = from\ 0\ to\ mTT_t.$$

Preferably, an additional constraint is defined by forcing the mean transit time $mTT_2$ of the second bolus function $B_2(t)$ to be higher than the time to peak $TP$ of the first bolus function $B_1(t)$ (denoted with $TP_1$):

$$mTT_2 > TP_1.$$

This additional constraint ensures that the second time-intensity function is always delayed with respect to the first time-intensity function.

The sets of constraints of the combined bolus function $B_c(t)$ so obtained for the different locations are stored into a repository 757 (action "A10.Constrain").

At this point, the modeler 721 fits each data array from the repository 718 by an instance of the combined bolus function $B_c(t)$, so as to obtain the corresponding combined time-intensity function (being defined by the values of its fitting parameters $O_c$, $A_1$, $m_1$, $s_1$, $A_2$, $m_2$, $s_2$ and $\Delta t$). For this purpose, the fitting parameters $A_1$, $m_1$, $s_1$, $A_2$, $m_2$, $s_2$ and $\Delta t$ are initialized as indicated in the repository 751, and they are constrained to vary (during the fitting operation) as indicated in the repository 757. The sets of fitting parameter values $O_c$, $A_1$, $m_1$, $s_1$, $A_2$, $m_2$, $s_2$ and $\Delta t$ so obtained for the different locations are stored into a repository 760 (action "A11.Combined fit").

Optionally, a differentiator 763 accesses both the repository 718 of the data arrays and the repository 760 of the fitting parameter values of the combined time-intensity functions. For each location, the differentiator 763 calculates a fitting array by evaluating the combined time-intensity function at every acquisition instant of the video images 706 (each one equal to a number of the corresponding video image 706 multiplied by the acquisition period); a difference array is then calculated by subtracting the fitting array from the data array 718 value by value. The difference arrays for the different locations are stored into a repository 766 (action "A12.Differentiate").

The modeler 721 then fits each difference array 766 by an instance of the simple bolus function $B_s(t)$, so as to obtain a third time-intensity function (being defined by the values of its fitting parameters $O_s$, $A_s$, $m_s$, and $s_s$, referred to as third fitting parameter values $O_3$, $A_3$, $m_3$, and $s_3$). The sets of third fitting parameter values so obtained for the different locations are stored into a repository 769 (action "A13.Difference fit"). These third time-intensity functions then represent a third passage of the contrast agent through the body-part (relating to a non-specific late phase thereof).

At this point, the sets of fitting parameter values 760 defining the combined time-intensity functions (including the sets of first fitting parameter values defining the first time-intensity functions and the sets of second fitting parameter values defining the second time-intensity functions that represent the first passage and the second passage, respectively, of the contrast agent through the corresponding locations of the body-part), and the possible sets of third fitting parameter values 769 defining the third time-intensity functions (that represent the third passage of the contrast agent through the same locations of the body-part) are supplied to a post-processor 772 (action "A14.Post-process"). For example, when the analysis process is performed at the level of pixels or groups of pixels, the post processor 772 generates a parametric image by assigning the value of a desired perfusion parameter to each pixel or group of pixel (which perfusion parameter values are calculated from one of the corresponding sets of first, second or third fitting parameter values); typically, the parametric image is color coded, by converting each perfusion parameter value into a discrete level (possibly applying a gain factor), and then associating it with the representation of a corresponding color (for example, by means of an index for accessing a location within a palette). Preferably, the parametric image is also superimposed on a selected one of the video images 706 (after restoring its full size by an interpolation operation when the parametric image has been calculated at the level of groups of pixels). Alternatively, when the analysis process is performed at the level of region of interest, the value of one or more perfusion parameters is calculated from one of the sets of first, second or third fitting parameter values. In any case, the information so obtained is used to characterize the corresponding locations of the body-part (for example, to detect and identify lesions of the body-part).

Modifications

Naturally, in order to satisfy local and specific requirements, a person skilled in the art may apply to the solution described above many logical and/or physical modifications and alterations. More specifically, although this solution has been described with a certain degree of particularity with reference to one or more embodiments thereof, it should be understood that various omissions, substitutions and changes in the form and details as well as other embodiments are possible. Particularly, different embodiments of the invention may even be practiced without the specific details (such as the numerical examples) set forth in the preceding description to provide a more thorough understanding thereof; conversely, well-known features may have been omitted or simplified in order not to obscure the description with unnecessary particulars. Moreover, it is expressly intended that specific elements and/or method steps described in connection with any embodiment of the disclosed solution may be incorporated in any other embodiment as a matter of general design choice.

First of all, it should be noted that the proposed data-processing method may be implemented independently of any interaction with the patient (and particularly with the contrast agent that may be pre-administered thereto before performing the method). Moreover, the contrast agent may also be administered to the patient in a non invasive manner, or in any case without any substantial physical intervention thereon that would require professional medical expertise or entail any health risk for the patient. Although the proposed method facilitates the task of a physician, it generally only provides intermediate results that may help him/her in examining the body-part - for example, for diagnostic purposes (even though the diagnosis for curative purposes *stricto sensu* is always made by the physician himself/herself).

In any case, similar considerations apply if the same solution is implemented with an equivalent data-processing method (by using similar steps with the same functions of more steps or portions thereof, removing some steps being non-essential, or adding further optional steps); moreover, the steps may be performed in a different order, concurrently or in an interleaved way (at least in part).For example, the proposed solution also lends itself to be put into practice with equivalent contrast agents; moreover, the contrast agent may be administered in an intra-arterial, intralymphatic, subcutaneous, intramuscular, intradermal, intraperitoneal, interstitial, intrathecal or intratumoral way, orally (for example, for imaging the gastro-intestinal tract), via a nebulizer into the airways, and the like. In any case, the possibility of applying the proposed solution to any other diagnostic systems - for example, based on Magnetic Resonance Imaging (MRI) or X-ray Computed Tomography (CT), is not excluded.

Moreover, nothing prevents applying the proposed solution to 3-D video images (at the level of voxels, groups of voxels or 3-D region of interest); in any case, the same solution may be applied either to a selected portion of the video images or to the whole extent thereof. The video images (or any equivalent input signals) to be processed according to the proposed solution may be provided with any other technique. For example, one or more of the above-described pre-processing operations may be omitted, and/or the video images may be subject to alternative or different pre-processing operations - for example, by discarding unsuitable video images, realigning the video images, or filtering the echo signals digitally (such as by mean, median, or low-pass Butterworth filters); likewise, the resulting time-intensity functions may be subject to different and/or alternative post-processing operations (for example, by discarding the pixels that do not provide an acceptable level of quality of the fitting operation).

Moreover, one or more of the above-mentioned time-intensity functions may be used in any other way; for example, information relating to a vascular morphology of the body-part (e.g., a shunting being present in tumor tissue but absent in normal tissue) may be extracted from the second time-intensity functions. Moreover, the same information may also be used for representing an animated perfusion of the body-part (as described in WO-A-2006/108868).

The same solution may also be applied to any other parametric functions, with whatever kind and number of fitting parameters.

Moreover, nothing prevents using different parametric functions for the initial fitting on the echo signals (for determining the initial time-to-peak values), for fitting the truncated echo signals (for initializing the fitting parameters of the combined bolus function), and/or for fitting the difference data arrays (for determining the third time-intensity functions).

In any case, the possibility of using any other parametric function to model the second passage of the contrast agent is not excluded (even when the parametric function is not based on the sum of multiple simple bolus functions). Moreover, the combined bolus function may be based on the sum of three or more simple bolus functions (for corresponding secondary passages of the contrast agent through the body-part).

Alternative algorithms may be used during the different fitting operations. For example, during the fitting of each echo signal by the combined bolus function it is possible to assign lower weights to a first portion of the echo signal (for example, in the same truncation interval) and higher weights to a remaining portion thereof - so as to increase the relative contribution of the portion of the echo signal in which the second passage appears; particularly, when the fitting operation is based on the above-mentioned gradient descent algorithm, the difference between the current instance of the combined bolus function and the echo signal (being used to determine the direction of the lowest gradient) may be based on a weighted sum of the instantaneous squared differences between each value of the time-intensity function and the corresponding echo power value according to their weights. Moreover, the proposed procedure may be reiterated by using the fitting parameter values of the combined time-intensity function to initialize the combined bolus function again for a further fitting thereof on the same echo signal.

Nothing prevents initializing the fitting parameters of the combined bolus function according to whatever combination of values being derived from the corresponding initial time-intensity function (in addition or in alternative to the truncated mean transit time value).

Particularly, the delay of each second bolus function may be initialized in any other way - for example, according to the corresponding initial time-to-peak value.

Moreover, the delay of each second bolus function may be initialized to a different multiple of the truncated mean transit time value (even equal to or higher than 1).

The mean transit time values of the first and second bolus functions may be calculated in another way from the truncated mean transit time value (for initializing one or more of their fitting parameters in whatever way according to the corresponding values calculated therefrom).

For example, the mean transit time values of the first and second bolus functions may be set to the truncated mean transit time value multiplied by different factors.

Similar considerations apply to other simple bolus functions (for example, lagged lognormal, gamma variate, local density random walk, and so on).

Likewise, one or more fitting parameters of the first and second bolus functions may be initialized to the corresponding truncated parameter values multiplied by different factors (either equal to or different from 1).

The fitting parameters of the combined bolus function may be constrained in any other way (even if this step is not strictly necessary).

Similar considerations apply if the mean of the combined bolus function is initialized and/or constrained according to the value of the same fitting parameter of the corresponding truncated time-intensity function, or *vice-versa* if other fitting parameters are initialized and/or constrained according to the corresponding truncated mean transit time value.

Moreover, it is possible to constrain the fitting parameters of the combined bolus function according to different multiples of the corresponding truncated fitting parameter values; for example, the constraint of each fitting parameter of the combined bolus function may be expressed as a percentage of allowed variation around its initialization value. In a different embodiment, it is also possible to apply this kind of constraining to all the fitting parameters of the combined bolus function.

Similar considerations apply to the constraining based on the mean transit time values of the first and second bolus functions.

Moreover, the mean transit time of the second bolus function may be constrained to exceed any other function of the time to peak of the first bolus function (even if this additional constraint may be omitted in a simplified implementation).

Similar considerations apply to the fitting parameters of the first and/or second bolus function that are initialized according to the corresponding truncated fitting parameter values.

The above-described solution lends itself to be implemented by estimating the peak instant of each echo signal in a different way; for example, it is possible to filter each echo signal by applying a Maximum Intensity Projection (MIP) algorithm (which holds the echo signal at its maximum over time), and then monitoring the filtered echo signal so obtained to detect its peak instant as soon as the filtered echo signal remains constant for a predefined stability time-window (as described in WO-A-2010/058014).

Naturally, the differentiation between the echo signals and the corresponding combined time-intensity functions (for determining the third time-intensity functions) is merely optional.

Each truncation interval may be determined in any other way (for example, by taking into account further values being derived from the initial time-intensity function in addition to the initial time-to-peak value); moreover, it is also possible to set the start of the truncation interval to an arrival instant, which is determined as the instant at which the echo signal exceeds a predefined threshold value. In any case, the above-described numerical examples of the factor to be applied to the initial time-to-peak values for obtaining the first passage times are merely illustrative.

The same solution may be implemented as a stand-alone module, as a plug-in for a control program of the ultrasound scanner, or even directly in the control program itself; it would be readily apparent that it is also possible to deploy the same solution as a service that is accessed through a network (such as in the Internet). Similar considerations apply if the program (which may be used to implement each embodiment of the invention) is structured in a different way, or if additional modules or functions are provided; likewise, the memory structures may be of other types, or may be replaced with equivalent entities (not necessarily consisting of physical storage media). In any case, the program may take any form suitable to be used by any data-processing system or in connection therewith (for example, within a virtual machine); particularly, the program may be in the form of external or resident software, firmware, or microcode (either in object code or in source code - for example, to be compiled or interpreted). Moreover, it is possible to provide the program on any computer-usable medium; the medium may be any element suitable to contain, store, communicate, propagate, or transfer the program. For example, the medium may be of the electronic, magnetic, optical, electromagnetic, infrared, or semiconductor type; examples of such medium are fixed disks (where the program can be pre-loaded), removable disks, tapes, cards, wires, fibers, wireless connections, networks, broadcast waves, and the like. In any case, the solution according to an embodiment of the present invention lends itself to be implemented even with a hardware structure (for example, integrated in a chip of semiconductor material), or with a combination of software and hardware.

Similar considerations apply if the ultrasound scanner has a different structure or includes equivalent components, or it has other operative characteristics (for example, with an imaging probe of the linear-, convex-, phased-, or matrix- array type). In any case, every component thereof may be separated into more elements, or two or more components may be combined together into a singe element; moreover, each component may be replicated to support the execution of the corresponding operations in parallel. It is also pointed out that (unless specified otherwise) any interaction between different components generally does not need to be continuous, and it may be either direct or indirect through one or more intermediaries. Alternatively, the same solution may be applied in a diagnostic system including an ultrasound scanner and a distinct computer (or any equivalent data-processing system); in this case, the recorded information is transferred from the ultrasound scanner to the computer for its processing (for example, through a digital, analogue or network connection).

The above-described solution, as well as any modification thereof, may advantageously be used in a conventional diagnostic method. More generally, the same solution may find application in any kind of diagnostic applications (in the broadest meaning of the term - for example, aimed at either discovering new lesions or monitoring known lesions) and for analyzing any kind of body-parts (for example, liver, prostate, heart, and so on).

**Claims**

1. A data-processing method (A1-A14) for analyzing a body-part (150) perfused with a contrast agent, the contrast agent being pre-administered as a bolus to circulate through the body-part with a first passage and possibly with at least one second passage during an analysis interval, wherein the method includes the steps of:

   providing (A1-A3) at least one input signal indicative of a response to an interrogation signal of a corresponding location of the body-part during the analysis interval, and
   fitting (A4-A11) each input signal over the analysis interval by an instance of a combined bolus function of time, based on a combination of a first simple bolus function of time modeling the first passage of the contrast agent and at least one second simple bolus function of time each one modeling a corresponding second passage of the contrast agent, being defined by the values of a set of first fitting parameters of the first simple bolus function, a set of second fitting parameters of each second simple bolus function and a delay parameter of each second simple bolus function with respect to the first simple bolus function, wherein the step of fitting each input signal includes:

      estimating (A4-A5) a peak instant of the input signal when the corresponding response reaches an absolute peak,
      setting (A6) a truncation interval within the analysis interval according to the peak instant,
      fitting (A7-A8) a truncated signal defined by the input signal over the truncation interval by an instance of a truncated simple bolus function of time, modeling a single passage of the contrast agent during the truncation interval, being defined by the values of a set of truncated fitting parameters, and
      initializing (A9) the first fitting parameters, the second fitting parameters of each second simple bolus function and the delay parameter of each second simple bolus function according to the values of the truncated fitting parameters, the step of initializing (A9) including:

         calculating (A9) the value of a truncated mean transit time of the truncated simple bolus function, and
         initializing (A9) at least one fitting parameter among the first fitting parameters, the second fitting parameters of each second simple bolus function and the delay parameter of each second simple bolus function according to the value of the truncated mean transit time.

2. The method (A1-A14) according to claim 1, wherein the step of initializing (A9) includes:

   initializing (A9) the delay parameter of each second simple bolus function to a fraction of the value of the truncated mean transit time.

3. The method (A1-A14) according to claim 2, wherein the step of initializing (A9) includes:

   calculating (A9) the value of a first mean transit time for the first simple bolus function and the value of a second mean transit time for each second simple bolus function according to the value of the truncated mean transit time, and
   initializing (A9) at least one of the first fitting parameters to the corresponding value calculated from the value of the first mean transit time, and at least one of the second fitting parameters of each second simple bolus function to the corresponding value calculated from the value of the second mean transit time.

4. The method (A1-A14) according to claim 3, wherein the step of initializing (A9) includes:

setting (A9) the value of the first mean transit time to the value of the truncated mean transit time multiplied by a first setting factor, and setting the value of the second mean transit time for each second simple bolus function to the value of the truncated mean transit time multiplied by a second setting factor.

5. The method (A1-A14) according to claim 3 or 4, wherein the first simple bolus function and each second simple bolus function are lognormal distribution functions, said at least one of the first fitting parameters and said at least one of the second fitting parameters including a mean and a standard deviation of a distribution of the natural logarithm of time of the first simple bolus function and the second simple bolus function, respectively.

6. The method (A1-A14) according to any claim from 1 to 5, wherein the step of initializing (A9) includes:

initializing (A9) at least a further one of the second fitting parameters of each second simple bolus function to the value of the corresponding truncated fitting parameter multiplied by an initialization factor different from 1.

7. The method (A1-A14) according to any claim from 1 to 6, wherein the step of fitting (A4-A11) each input signal further includes:

constraining (A10) said at least one fitting parameter according to the value of the truncated mean transit time.

8. The method (A1-A14) according to claim 7, wherein the step of constraining (A10) includes:

constraining (A10) the delay parameter of each second simple bolus function to range between a lower delay limit and an upper delay limit, the lower delay limit and the upper delay limit being equal to the value of the truncated mean transit time multiplied by a lower delay constraining factor and an upper delay constraining factor, respectively.

9. The method (A1-A14) according to claim 7 or 8 when dependent directly or indirectly on claim 3, wherein the step of constraining (A10) includes:

calculating (A10) a lower first mean transit time limit and an upper first mean transit time limit for the first simple bolus function equal to the value of the truncated mean transit time multiplied by a lower first constraining factor and an upper first constraining factor, respectively, and a lower second mean transit time limit and an upper second mean transit time limit for each second simple bolus function equal to the value of the truncated mean transit time multiplied by a lower second constraining factor and an upper second constraining factor, respectively, and
constraining (A10) said at least one of the first fitting parameters to range between a corresponding lower first limit and a corresponding upper first limit calculated from the lower first mean transit time limit and the upper first mean transit time limit, respectively, and constraining said at least one of the second fitting parameters to range between a corresponding lower second limit and a corresponding upper second limit calculated from the lower second mean transit time limit and the upper second mean transit time limit, respectively.

10. The method (A1-A14) according to any claim from 7 to 9, wherein the step of constraining (A10) includes:

constraining (A10) a second mean transit time of each second simple bolus function to exceed a first time to peak of the first simple bolus function.

11. The method (A1-A14) according to any claim from 7 to 10, wherein the step of constraining (A10) includes:

constraining (A10) at least a further one of the first fitting parameters to range between a lower further first limit and an upper further first limit equal to the value of the corresponding truncated parameter multiplied by a lower further first constraining factor and an upper further first constraining factor, respectively, and at least a further one of the second fitting parameters of each second simple bolus function to range between a lower further second limit and an upper further second limit equal to the value of the corresponding truncated parameter multiplied by a lower further second constraining factor and an upper further second constraining factor, respectively.

**12.** The method (A1-A14) according to any claim from 1 to 11, wherein the step for estimating (A4-A5) a peak instant includes:

fitting (A4) the input signal over the analysis interval by an instance of an initial simple bolus function of time modeling a single passage of the contrast agent during the analysis interval, and
calculating (A5) the peak instant from the initial simple bolus function.

**13.** The method (A1-A14) according to any claim from 1 to 12, further including the steps of:

calculating (A12) a difference signal from each input signal by subtracting the values of the corresponding combined bolus function at corresponding instants from the input signal, and
fitting (A13) each difference signal by a third simple bolus function of time modeling a third passage of the contrast agent.

**14.** A computer program (700) including code means for causing a data-processing system (100) to perform the steps of the data-processing method according to any claim from 1 to 13 when the computer program is executed on the data-processing system.

**15.** A diagnostic system (100) including means (700) for performing the steps of the data-processing method (A1-A14) according to any claim from 1 to 13.


**Patentansprüche**

**1.** Datenverarbeitungsverfahren (A1-A14) zum Analysieren eines Körperteils (150), der mit einem Kontrastmittel perfundiert wird, wobei das Kontrastmittel vorab als Bolus verabreicht wird, um während eines Analyseintervalls mit einer ersten Passage und möglicherweise wenigstens einer zweiten Passage durch den Körperteil zu zirkulieren, wobei das Verfahren folgende Schritte beinhaltet:

Bereitstellen (A1-A3) wenigstens eines Eingangssignals, das für eine Reaktion auf ein Abfragesignal einer entsprechenden Stelle des Körperteils während des Analyseintervalls indikativ ist, und
Anpassen (A4-A11) jedes Eingangssignals über das Analyseintervall durch eine Instanz einer kombinierten Bolusfunktion von Zeit, die auf einer Kombination einer ersten einfachen Bolusfunktion von Zeit, die die ersten Passage des Kontrastmittels abbildet, und wenigstens einer zweiten einfachen Bolusfunktion von Zeit, die je eine entsprechende zweite Passage des Kontrastmittels abbildet, basiert und durch die Werte eines Satzes von ersten Anpassungsparametern der ersten einfachen Bolusfunktion, eines Satzes von zweiten Anpassungsparametern jeder zweiten einfachen Bolusfunktion und eines Verzögerungsparameters jeder zweiten einfachen Bolusfunktion bezüglich der ersten einfachen Bolusfunktion definiert wird, wobei der Schritt des Anpassens jedes Eingangssignals beinhaltet:

Schätzen (A4-A5) einer Spitzeninstanz des Eingangssignals, wenn die entsprechende Reaktion eine absolute Spitze erreicht,
Einstellen (A6) eines Beschneidungsintervalls innerhalb des Analyseintervalls entsprechend der Spitzeninstanz,
Anpassen (A7-A8) eines beschnittenen Signals, das durch das Eingangssignal über das Beschneidungsintervall definiert wird, durch eine Instanz einer beschnittenen einfachen Bolusfunktion von Zeit, die eine einzelne Passage des Kontrastmittels während des Beschneidungsintervalls abbildet und durch die Werte eines Satzes von beschnittenen Anpassungsparametern definiert wird, und
Initialisieren (A9) der ersten Anpassungsparameter, der zweiten Anpassungsparameter jeder zweiten einfachen Bolusfunktion und des Verzögerungsparameters jeder zweiten einfachen Bolusfunktion entsprechend den Werten der beschnittenen Anpassungsparameter, wobei der Schritt des Initialisierens (A9) beinhaltet:

Berechnen (A9) des Wertes einer beschnittenen mittleren Durchgangszeit der beschnittenen einfachen Bolusfunktion, und
Initialisieren (A9) wenigstens eines Anpassungsparameters von den ersten Anpassungsparametern, den zweiten Anpassungsparametern jeder zweiten einfachen Bolusfunktion und dem Verzögerungsparameter jeder zweiten einfachen Bolusfunktion entsprechend dem Wert der beschnittenen mittleren Durchgangszeit.

2. Verfahren (A1-A14) nach Anspruch 1, wobei der Schritt des Initialisierens (A9) beinhaltet:

Initialisieren (A9) des Verzögerungsparameters jeder zweiten einfachen Bolusfunktion auf einen Bruchteil des Wertes der beschnittenen mittleren Durchgangszeit.

3. Verfahren (A1-A14) nach Anspruch 2, wobei der Schritt des Initialisierens (A9) beinhaltet:

Berechnen (A9) des Wertes einer ersten mittleren Durchgangszeit für die erste einfache Bolusfunktion und des Wertes einer zweiten mittleren Durchgangszeit für jede zweite einfache Bolusfunktion entsprechend dem Wert der beschnittenen mittleren Durchgangszeit, und
Initialisieren (A9) wenigstens eines der ersten Anpassungsparameter auf den entsprechenden aus dem Wert der ersten mittleren Durchgangszeit berechneten Wert und wenigstens eines der zweiten Anpassungsparameter jeder zweiten einfachen Bolusfunktion auf den entsprechenden aus dem Wert der zweiten mittleren Durchgangszeit berechneten Wert.

4. Verfahren (A1-A14) nach Anspruch 3, wobei der Schritt des Initialisierens (A9) beinhaltet:

Einstellen (A9) des Wertes der ersten mittleren Durchgangszeit auf den Wert der beschnittenen mittleren Durchgangszeit multipliziert mit einem ersten Einstellungsfaktor und Einstellen des Wertes der zweiten mittleren Durchgangszeit für jede zweite einfache Bolusfunktion auf den Wert der beschnittenen mittleren Durchgangszeit multipliziert mit einem zweiten Einstellungsfaktor.

5. Verfahren (A1-A14) nach Anspruch 3 oder 4, wobei die erste einfache Bolusfunktion und jede zweite einfache Bolusfunktion Lognormalverteilungsfunktionen sind, wobei der wenigstens eine der ersten Anpassungsparameter und der wenigstens eine der zweiten Anpassungsparameter eine mittlere Abweichung und eine Standardabweichung einer Verteilung des natürlichen Zeitlogarithmus der ersten einfachen Bolusfunktion bzw. der zweiten einfachen Bolusfunktion umfassen.

6. Verfahren (A1-A14) nach einem der Ansprüche 1 bis 5, wobei der Schritt des Initialisierens (A9) beinhaltet:

Initialisieren (A9) wenigstens eines weiteren der zweiten Anpassungsparameter jeder zweiten einfachen Bolusfunktion auf den Wert des entsprechenden beschnittenen Anpassungsparameters multipliziert mit einem Initialisierungsfaktor, der nicht 1 ist.

7. Verfahren (A1-A14) nach einem der Ansprüche 1 bis 6, wobei der Schritt des Anpassens (A4-A11) jedes Eingangssignals ferner beinhaltet:

Beschränken (A10) des wenigstens einen Anpassungsparameters entsprechend dem Wert der beschnittenen mittleren Durchgangszeit.

8. Verfahren (A1-A14) nach Anspruch 7, wobei der Schritt des Beschränkens (A10) beinhaltet:

derartiges Beschränken (A10) des Verzögerungsparameters jeder zweiten einfachen Bolusfunktion, dass er zwischen einer unteren Verzögerungsgrenze und einer oberen Verzögerungsgrenze liegt, wobei die untere Verzögerungsgrenze und die obere Verzögerungsgrenze gleich dem Wert der beschnittenen mittleren Durchgangszeit multipliziert mit einem Faktor der unteren Verzögerungsbeschränkung bzw. einem Faktor der oberen Verzögerungsbeschränkung sind.

9. Verfahren (A1-A14) nach Anspruch 7 oder 8, wenn direkt oder indirekt abhängig von Anspruch 3, wobei der Schritt des Beschränkens (A10) beinhaltet:

Berechnen (A10) einer unteren Grenze der ersten mittleren Durchgangszeit und einer oberen Grenze der ersten mittleren Durchgangszeit für die erste einfache Bolusfunktion, die gleich dem Wert der beschnittenen mittleren Durchgangszeit multipliziert mit einem unteren ersten Beschränkungsfaktor bzw. einem oberen ersten Beschränkungsfaktor sind, und einer unteren Grenze der zweiten mittleren Durchgangszeit und einer oberen Grenze der zweiten mittleren Durchgangszeit für jede zweite einfache Bolusfunktion, die gleich dem Wert der beschnittenen mittleren Durchgangszeit multipliziert mit einem unteren zweiten Beschränkungsfaktor bzw. einem oberen zweiten Beschränkungsfaktor sind, und

derartiges Beschränken (A10) des wenigstens einen der ersten Anpassungsparameter, dass er zwischen einer entsprechenden unteren ersten Grenze und einer entsprechenden obereren ersten Grenze liegt, die aus der unteren Grenze der ersten mittleren Durchgangszeit bzw. der oberen Grenze der ersten mittleren Durchgangszeit berechnet werden, und derartiges Beschränken des wenigstens einen der zweiten Anpassungsparameter, dass er zwischen einer entsprechenden unteren zweiten Grenze und einer entsprechenden oberen zweiten Grenze liegt, die aus der unteren Grenze der zweiten mittleren Durchgangszeit bzw. der oberen Grenze der zweiten mittleren Durchgangszeitlimit berechnet werden.

**10.** Verfahren (A1-A14) nach einem der Ansprüche 7 bis 9, wobei der Schritt des Beschränkens (A10) beinhaltet:

derartiges Beschränken (A10) einer zweiten mittleren Durchgangszeit jeder zweiten einfachen Bolusfunktion, dass sie eine erste Zeit bis zur Spitze der ersten einfachen Bolusfunktion überschreitet.

**11.** Verfahren (A1-A14) nach einem der Ansprüche 7 bis 10, wobei der Schritt des Beschränkens (A10) beinhaltet:

derartiges Beschränken (A10) wenigstens eines weiteren der ersten Anpassungsparameter, dass er zwischen einer weiteren unteren ersten Grenze und einer oberen weiteren ersten Grenze liegt, die gleich dem Wert des entsprechenden beschnittenen Parameters multipliziert mit einem unteren weiteren ersten Beschränkungsfaktor bzw. einem oberen weiteren ersten Beschränkungsfaktor sind, und wenigstens eines weiteren der zweiten Anpassungsparameter jeder zweiten einfachen Bolusfunktion, so dass er zwischen einer unteren weiteren zweiten Grenze und einer oberen weiteren zweiten Grenze liegt, die gleich dem Wert des entsprechenden beschnittenen Parameters multipliziert mit einem weiteren unteren zweiten Beschränkungsfaktor bzw. einem oberen weiteren Beschränkungsfaktor sind.

**12.** Verfahren (A1-A14) nach einem der Ansprüche 1 bis 11, wobei der Schritt des Schätzens (A4-A5) einer Spitzeninstanz beinhaltet:

Anpassen (A4) des Eingangssignals über das Analyseintervall durch eine Instanz einer anfänglichen einfachen Bolusfunktion von Zeit, die eine einzelne Passage des Kontrastmittels während des Analyseintervalls abbildet, und
Berechnen (A5) der Spitzeninstanz der anfänglichen einfachen Bolusfunktion.

**13.** Verfahren (A1-A14) nach einem der Ansprüche 1 bis 12, das ferner folgende Schritte beinhaltet:

Berechnen (A12) eines Differenzsignals aus jedem Eingangssignal durch Subtrahieren der Werte der entsprechenden kombinierten Bolusfunktion an entsprechenden Instanzen von dem Eingangssignal, und
Anpassen (A13) jedes Differenzsignals durch eine dritte einfache Bolusfunktion von Zeit, die eine dritte Passage des Kontrastmittels abbildet.

**14.** Computerprogramm (700), aufweisend eine Code-Einrichtung, um zu bewirken, dass ein Datenverarbeitungssystem (100) die Schritte des Datenverarbeitungsverfahrens nach einem der Ansprüche 1 bis 13 ausführt, wenn das Computerprogramm auf dem Datenverarbeitungssystem ausgeführt wird.

**15.** Diagnostisches System (100), aufweisend eine Einrichtung (700) zum Ausführen der Schritte des Datenverarbeitungsverfahrens (A1-A14) nach einem der Ansprüche 1 bis 13.

**Revendications**

**1.** Procédé de traitement de données (A1-A14) pour analyser une partie d'un corps (150) perfusée avec un agent de contraste, l'agent de contraste étant pré-administré sous forme d'un bolus pour circuler dans la partie du corps avec un premier passage et éventuellement avec au moins un deuxième passage pendant un intervalle d'analyse, le procédé comprenant les étapes suivantes :

fournir (A1-A3) au moins un signal d'entrée indicatif d'une réponse à un signal d'interrogation d'un emplacement correspondant de la partie du corps pendant l'intervalle d'analyse, et

ajuster (A4-A11) chaque signal d'entrée sur l'intervalle d'analyse par une instance d'une fonction du temps d'un bolus combinée, sur la base d'une combinaison d'une première fonction du temps d'un bolus simple modélisant le premier passage de l'agent de contraste et d'au moins une deuxième fonction du temps d'un bolus simple, chacune d'elles modélisant un deuxième passage correspondant de l'agent de contraste, définie par les valeurs d'un ensemble de premiers paramètres d'ajustement de la première fonction de bolus simple, d'un ensemble de deuxièmes paramètres d'ajustement de chaque deuxième fonction de bolus simple et d'un paramètre de retard de chaque deuxième fonction de bolus simple par rapport à la première fonction de bolus simple, l'étape d'ajustement de chaque signal d'entrée comprenant :

estimer (A4-A5) un instant de pic du signal d'entrée lorsque la réponse correspondante atteint un pic absolu,
régler (A6) un intervalle de troncature dans l'intervalle d'analyse conformément à l'instant du pic,
ajuster (A7-A8) un signal tronqué défini par le signal d'entrée sur l'intervalle de troncature par une instance d'une fonction du temps d'un bolus simple tronquée, modélisant un unique passage de l'agent de contraste pendant l'intervalle de troncature, définie par les valeurs d'un ensemble de paramètres d'ajustement tronqué, et
initialiser (A9) les premiers paramètres d'ajustement, les deuxièmes paramètres d'ajustement de chaque deuxième fonction de bolus simple et le paramètre de retard de chaque deuxième fonction de bolus simple conformément aux valeurs des paramètres d'ajustement tronqués, l'étape d'initialisation (A9) comprenant :

calculer (A9) la valeur d'un temps de transit moyen tronqué de la fonction de bolus simple tronquée, et initialiser (A9) au moins un paramètre d'ajustement parmi les premiers paramètres d'ajustement, le deuxième paramètre d'ajustement de chaque deuxième fonction de bolus simple et le paramètre de retard de chaque deuxième fonction de bolus simple conformément à la valeur du temps de transit moyen tronqué.

2. Procédé (A1-A14) selon la revendication 1, dans lequel l'étape d'initialisation (A9) comprend :

initialiser (A9) le paramètre de retard de chaque deuxième fonction de bolus simple à une fraction de la valeur du temps de transit moyen tronqué.

3. Procédé (A1-A14) selon la revendication 2, dans lequel l'étape d'initialisation (A9) comprend :

calculer (A9) la valeur d'un premier temps de transit moyen pour la première fonction de bolus simple et la valeur d'un deuxième temps de transit moyen pour chaque deuxième fonction de bolus simple conformément à la valeur du temps de transit moyen tronqué, et
initialiser (A9) au moins l'un des premiers paramètres d'ajustement à la valeur correspondante calculée à partir de la valeur du premier temps de transit moyen, et au moins l'un des deuxièmes paramètres d'ajustement de chaque deuxième fonction de bolus simple à la valeur correspondante calculée à partir de la valeur du deuxième temps de transit moyen.

4. Procédé (A1-A14) selon la revendication 3, dans lequel l'étape d'initialisation (A9) comprend :

régler (A9)) la valeur du premier temps de transit moyen à la valeur du temps de transit moyen tronqué multipliée par un premier facteur de réglage, et régler la valeur du deuxième temps de transit moyen pour chaque deuxième fonction de bolus simple à la valeur du temps de transit moyen tronqué multipliée par un deuxième facteur de réglage.

5. Procédé (A1-A14) selon la revendication 3 ou 4, dans lequel la première fonction de bolus simple et chaque deuxième fonction de bolus simple sont des fonctions à distribution log-normale, ledit au moins l'un des premiers paramètres d'ajustement et ledit au moins l'un des deuxièmes paramètres d'ajustement comprenant une moyenne et un écart type d'une distribution logarithmique naturelle du temps de la première fonction de bolus simple et de la deuxième fonction de bolus simple, respectivement.

6. Procédé (A1-A14) selon l'une quelconque des revendications 1 à 5, dans lequel l'étape d'initialisation (A9) comprend :

initialiser (A9) au moins un autre des deuxièmes paramètres d'ajustement de chaque deuxième fonction de bolus simple à la valeur du paramètre d'ajustement tronqué correspondant multipliée par un facteur d'initialisation différent de 1.

**7.** Procédé (A1-A14) selon l'une quelconque des revendications 1 à 6, dans lequel l'étape d'ajustement (A4-A11) de chaque signal d'entrée comprend en outre :

contraindre (A10) ledit au moins un paramètre d'ajustement conformément à la valeur du temps de transit moyen tronqué.

**8.** Procédé (A1-A14) selon la revendication 7, dans lequel l'étape de contrainte (A10) comprend :

contraindre (A10) le paramètre de retard de chaque deuxième fonction de bolus simple à se trouver entre une limite de retard inférieure et une limite de retard supérieure, la limite de retard inférieure et la limite de retard supérieure étant égales à la valeur du temps de transit moyen tronqué multipliée par un facteur de contrainte de retard inférieur et un facteur de contrainte de retard supérieur, respectivement.

**9.** Procédé (A1-A14) selon la revendication 7 ou 8, dans leur dépendance directe ou indirecte de la revendication 3, dans lequel l'étape de contrainte (A10) comprend :

calculer (A10) une première limite inférieure du temps de transit moyen et une première limite supérieure du temps de transit moyen pour la première fonction de bolus simple, égales à la valeur du temps de transit moyen tronqué multipliée par un premier facteur de contrainte inférieur et un premier facteur de contrainte supérieur, respectivement, et une deuxième limite inférieure de temps de transit moyen et une deuxième limite supérieure de temps de transit moyen pour chaque deuxième fonction de bolus simple, égales à la valeur du temps de transit moyen tronqué multipliée par un deuxième facteur de contrainte inférieur et un deuxième facteur de contrainte supérieur, respectivement, et
contraindre (A10) ledit au moins l'un des premiers paramètres d'ajustement à se trouver entre une première limite inférieure correspondante et une première limite supérieure correspondante, calculées à partir de la première limite inférieure de temps de transit moyen et de la première limite supérieure de temps de transit moyen, respectivement, et contraindre ledit au moins l'un des deuxièmes paramètres d'ajustement à se trouver entre une deuxième limite inférieure correspondante et une deuxième limite supérieure correspondante, calculées à partir de la deuxième limite inférieure de temps de transit moyen et de la deuxième limite supérieure de temps de transit moyen, respectivement.

**10.** Procédé (A1-A14) selon l'une quelconque des revendications 7 à 9, dans lequel l'étape de contrainte (A10) comprend :

contraindre (A10) un deuxième temps de transit moyen de chaque deuxième fonction de bolus simple à dépasser un premier temps jusqu'au pic de la première fonction de bolus simple.

**11.** Procédé (A1-A14) selon l'une quelconque des revendications 7 à 10, dans lequel l'étape de contrainte (A10) comprend :

contraindre (A10) au moins un autre des premiers paramètres d'ajustement à se trouver entre une autre première limite inférieure et une autre première limite supérieure, égales à la valeur du paramètre tronqué correspondant multipliée par un autre premier facteur de contrainte inférieur et un autre premier facteur de contrainte supérieur, respectivement, et au moins un autre des deuxièmes paramètres d'ajustement de chaque deuxième fonction de bolus simple à se trouver entre une autre deuxième limite inférieure et une autre deuxième limite supérieure, égales à la valeur du paramètre tronqué correspondant multipliée par un autre deuxième facteur de contrainte inférieur et un autre deuxième facteur de contrainte supérieur, respectivement.

**12.** Procédé (A1-A14) selon l'une quelconque des revendications 1 à 11, dans lequel l'étape pour estimer (A4-A5) un instant de pic comprend :

ajuster (A4) le signal d'entrée sur l'intervalle d'analyse par une instance d'une fonction du temps de bolus simple initiale modélisant un unique passage de l'agent de contraste pendant l'intervalle d'analyse, et
calculer (A5) l'instant du pic à partir de la fonction de bolus simple initiale.

**13.** Procédé (A1-A14) selon l'une quelconque des revendications 1 à 12, comprenant en outre les étapes suivantes :

calculer (A12) un signal de différence à partir de chaque signal d'entrée en soustrayant du signal d'entrée les valeurs de la fonction de bolus combinée correspondante à des instants correspondants, et
ajuster (A13) chaque signal de différence par une troisième fonction du temps de bolus simple modélisant un troisième passage de l'agent de contraste.

**14.** Programme d'ordinateur (700) comprenant des moyens de code pour amener un système de traitement de données (100) à réaliser les étapes du procédé de traitement de données selon l'une quelconque des revendications 1 à 13, lorsque le programme d'ordinateur est exécuté sur le système de traitement de données.

**15.** Système de diagnostic (100) comprenant des moyens (700) pour réaliser les étapes du procédé de traitement de données (A1-A14) selon l'une quelconque des revendications 1 à 13.

<u>FIG.1</u>

Echo-power (a.u.)

Time (s)

FIG.2A

FIG.2B

Echo-power (a.u.)

Time (s)

## FIG.2C

Echo-power (a.u.)

Time (s)

## FIG.2D

FIG.2E

FIG.3A

FIG.3B

FIG.4A

FIG.4B

FIG.4C

FIG.5A

FIG.5B

FIG.5C

FIG.6A

FIG.6B

FIG.6C

FIG.7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006108868 A
- WO 2006067201 A
- WO 2009083557 A
- WO 2010058014 A
- US 6216094 B
- EP 0458745 A
- WO 9115244 A
- EP 0554213 A
- WO 9409829 A
- WO 9516467 A
- WO 2004110279 A

**Non-patent literature cited in the description**

- **MARTIN KRIX ; CHRISTIAN PLATHOW ; FABIAN KIESSLING ; FELIX HERTH ; ANDREAS KARCHER ; MARCO ESSIG ; HARRY SCHMITTECKERT ; HANS-ULRICH KAUCZOR ; STEFAN DELORME.** Quantification of perfusion of liver tissue and metastases using a multivessel model for replenishment kinetics of ultrasound contrast agents. *Ultrasound in Med. & Biol.,* vol. 30 (10), 1355-1363
- **R.A.F. LINTON ; N.W.F. LINTON ; D.M. BAND.** A new method of analyzing indicator dilution curves. *Cardiovascular Research,* 1995, vol. 30, 930-938
- **RAFTER et al.** Imaging technologies and techniques. *Cardiology Clinics,* 2004, vol. 22, 181-197